# EUROPÄISCHE PATENTSCHRIFT

(11) **EP 0 896 670 B1**
(45) Veröffentlichungstag und Bekanntmachung des Hinweises auf die Patenterteilung: **16.06.2004**
(21) Anmeldenummer: 97921792.4
(22) Anmeldetag: 26.04.1997
(51) Int. Cl.: G01N 33/18, G01N 31/00, G01N 1/00

(54) **VERFAHREN UND VORRICHTUNG ZUR BESTIMMUNG DES GEHALTS AN OXIDIERBAREN INHALTSSTOFFEN IN WÄSSRIGEN FLÜSSIGKEITEN**
PROCESS AND DEVICE FOR DETERMINING THE OXIDISABLE SUBSTANCE CONTENT OF AQUEOUS LIQUIDS
PROCEDE ET DISPOSITIF POUR LA DETERMINATION DE LA TENEUR EN SUBSTANCES OXYDABLES DE LIQUIDES AQUEUX

(30) Priorität: 03.05.1996 DE 19617910
(43) Veröffentlichungstag der Anmeldung: 17.02.1999
(73) Patentinhaber: ISCO Inc., Lincoln Nebraska 68501 (US)
(72) Erfinder: SIEPMANN, Friedrich, W., 64823 Gross-Umstadt (DE)
(74) Vertreter: Katscher, Helmut, Dipl.-Ing.
(86) Internationale Anmeldenummer: PCT/EP1997/002165
(87) Internationale Veröffentlichungsnummer: WO 1997/042499

(56) Entgegenhaltungen:
- DE-A- 2 407 604
- DE-C- 4 344 441
- GB-A- 2 016 688
- US-A- 3 567 387
- US-A- 4 089 209
- US-A- 5 158 894
- GIT FACHZEITSCRIFT FÜR DAS LABORATORIUM, Bd. 23, Nr. 8, August 1979, DE, Seiten 738-747, XP002039275 F.EHRENBERGER: "Zur Bestimmung von Sauerstoffbedarfs- und Kohlenstoff-Kennzahlen (TOD,TOC,DOC usw.) in der Wasserqualitätsbestimmung" in der Anmeldung erwähnt

## Beschreibung

Die Erfindung betrifft ein Verfahren zur Bestimmung des Gehalts an oxidierbaren Inhaltsstoffen in wässrigen Flüssigkeiten, bei dem Flüssigkeitsproben einem Verbrennungsofen zugeführt und thermisch behandelt werden und der Inhaltsstoff zu einem gasförmigem Oxid verbrannt wird und in einer Probe des so erhaltenen Abgases der Gehalt an gasförmigem Oxid durch Infrarotmessung ermittelt wird.

Ein wichtiges Anwendungsgebiet dieses Verfahrens ist die Bestimmung des Kohlenstoffgehalts und/oder des Stickstoffgehalts in Abwasser. Diese oxidierbaren Inhaltsstoffe werden im allgemeinen wie folgt bezeichnet:
- TC: (Total Carbon) der gesamte in der wässrigen Flüssigkeit enthaltene Kohlenstoff;
- TOC: (Total Organic Carbon) der gesamte in der wässrigen Flüssigkeit in Form von organischen Verbindungen enthaltene Kohlenstoff;
- TIC: (Total Inorganic Carbon) der gesamte in der wässrigen Flüssigkeit in Form von anorganischen Verbindungen enthaltene Kohlenstoff;
- TN: (Total Nitrogen) der gesamte in der wässrigen Flüssigkeit enthaltene Stickstoff.

In dem Aufsatz von F. Ehrenberger, "Zur Bestimmung von Sauerstoffbedarfs- und Kohlenstoff-Kennzahlen in der Wasserqualitätsbestimmung", GIT Fachz. Lab. 23 Jg 8/79, S. 738-747, werden verschiedene Methoden zur TOC-Bestimmung beschrieben, die auf der naßchemischen oder thermischen Umsetzung der organischen Inhaltsstoffe und der quantitativen Oxidation beruhen.

Bei den bekannten Verfahren der eingangs genannten Gattung durchläuft die Flüssigkeitsprobe einen Feinfilter, um Partikel zu entfernen, die größer als 100-200 µm sind. Die Flüssigkeitsprobe würde dann - im Falle der Bestimmung des TOC ggf. mit einer Zwischenbehandlung zum Entfernen der anorganischen Verbindungen - dem Verbrennungsofen zugeführt. Dort werden die organischen Inhaltsstoffe thermisch zu Kohlendioxid (CO₂) umgesetzt. Das entstandene Kohlendioxid wird mit Hilfe eines Transportgases, das in der Regel auch den nötigen Verbrennungssauerstoff liefert, durch einen Kühler mit Wasserabscheider, einen Gasfilter und eine Infrarot-Auswerteeinheit transportiert. Das bei der Verbrennung entstandene Kohlendioxid wird durch Infrarotmessung bestimmt, und aus diesem Wert wird der TOC errechnet.

Bei dem beschriebenen Verfahren ist das Probenvolumen je Zeiteinheit oder Einzelprobe mit 20-100 µl extrem gering. Das geringe Probenvolumen hat zur Folge, daß die zuführenden Leitungen ebenfalls einen extrem geringen Querschnitt haben müssen, um größere Zeitverluste zu vermeiden.

Ein weiteres bekanntes Verfahren (gwf-wasser/abwasser 120 (1979) H. 5) zur Bestimmung des TOC ohne den Einsatz eines Katalysators erfordert erhöhte Temperaturen (1100 bis 1200°C) und eine längere Aufenthaltszeit im Ofen. Eine einfache Verlängerung der Aufenthaltszeit im Ofen durch Vergrößerung des Ofenvolumens hat jedoch den Nachteil größerer Totzeiten. Der gleiche Effekt wird bei dem bekannten Verfahren dadurch angestrebt, daß die mit Trägergas vermischte Probe auf ihrem Weg durch den Verbrennungsofen mittels Umlenkblechen (Schikanen) mehrmals durch die heißeste Ofenzone geleitet wird, bevor sie den Ofen verläßt und der Infrarotmessung zur Auswertung zugeführt wird.

Die Probe wird mit Hilfe eines Transportgases (Carrier-Gas) und/oder einer feindüsigen Dosiervorrichtung in den Ofen eingebracht. Hierbei sind folgende Zusammenhänge wichtig:

Das Probenvolumen ist mit 20-100 ul pro Minute oder Einzelprobe extrem gering. Es muß zusammen mit dem Transportgas in den Verbrennungsofen eingespritzt werden. Das Transportgas hat vor allem die Aufgabe, das durch Verbrennung entstandene CO₂-Gas aus dem Verbrennungsofen bis zu einer Infrarot-Meßkammer zu transportieren. Die Transportgasmenge bestimmt die CO₂-Austauschzeit im Verbrennungsofen. Die Transportgasmenge verdünnt jedoch das Ergebnissignal des zu messenden CO₂ aus dem der TC, TOC usw. ermittelt wird.

Um unter den zuvor genannten Bedingungen einen guten Eintrag der Abwasserprobe in den Verbrennungsofen zu erreichen, muß eine mechanische Vorrichtung die Probe mit hoher Energie durch eine sehr dünne Düse in den Verbrennungsofen befördern oder die Flüssigkeitsprobe muß mit Hilfe des Transportgases durch eine sehr dünne Düse (Düse mit sehr kleinem Querschnitt) in den Verbrennungsofen gespritzt werden.

Die notwendige Einhaltung der oben genannten Bedingungen hat zur Folge, daß der Einsatz der Einspritzdüsen mit sehr geringem Durchmesser (50-250 um) sehr schnell zu Verstopfungen der Düse führt. Vergrößert man die Düse, muß man mehr Transportgas einsetzen, um das Gemisch mit ausreichender Energie in den Verbrennungsofen zu verdüsen. Dadurch wird das Ergebnissignal des CO₂ verringert.

Aufgabe der Erfindung ist es daher, ein Verfahren der eingangs genannten Art so auszugestalten, daß in einfacher Weise eine sehr kleine, aber genau bestimmbare Probenmenge in den Verbrennungsofen eingebracht werden kann, ohne daß hierfür ein Transportgas verwendet wird und/oder die Gefahr des Verstopfens von sehr dünnen Leitungen besteht.

Diese Aufgabe wird erfindungsgemäß dadurch gelöst, daß eine an einer Heizfläche anhängende Flüssigkeitsprobe bei geschlossenem Verbrennungsofen verdampft wird, um die der Infrarotmessung zuzuführende Abgasprobe zu erhalten, und daß der bei der Verdampfung entstehende Druckanstieg im Verbrennungsofen oder der Feuchteanstieg im Gas gemessen und daraus die Probenmenge rechnerisch bestimmt wird.

Die Absonderung einer sehr kleinen Probenmenge geschieht hierbei in sehr einfacher Weise dadurch, daß die nach dem Benetzen der Heizfläche mit der Flüssigkeit daran hängenbleibende Flüssigkeitsmenge die gewünschte, geringe Probenmenge bildet, deren Volumen aber noch genau bestimmt werden muß. Diese Volumenbestimmung erfolgt in sehr einfacher Weise mit hoher Genauigkeit dadurch, daß der bei der Verdampfung entstehende Druckanstieg oder der Feuchteanstieg gemessen wird. Dieser Druckanstieg oder Feuchteanstieg lässt rechnerisch in einfacher Weise auf die verdampfte Flüssigkeitsmenge rückschließen.

In besonders einfacher Weise erfolgt das Benetzen der Heizfläche dadurch, daß der Verbrennungsofen mit der zu untersuchenden Flüssigkeit mindestens teilweise gefüllt und anschließend durch Gaszufuhr entleert wird. Die Tatsache, daß dabei auch die Wände des Verbrennungsofens mit Flüssigkeit benetzt werden, beeinflusst das Meßergebnis nicht, weil die an den Wänden anhängende Flüssigkeitsmenge nicht verdampft wird und damit bei der Auswertung unberücksichtigt bleibt.

Eine erfindungsgemäße Vorrichtung zur Durchführung des Verfahrens, die einen Verbrennungsofen und eine nachgeschaltete Einrichtung zur Infrarotmessung aufweist, ist dadurch gekennzeichnet, daß der Verbrennungsofen eine abschließbare Ofenkammer aufweist, in die ein Heizelement ragt und an die eine Druckmesseinrichtung angeschlossen ist.

Weitere vorteilhafte Ausgestaltungen des Erfindungsgedankens sind Gegenstand weiterer abhängiger Ansprüche.

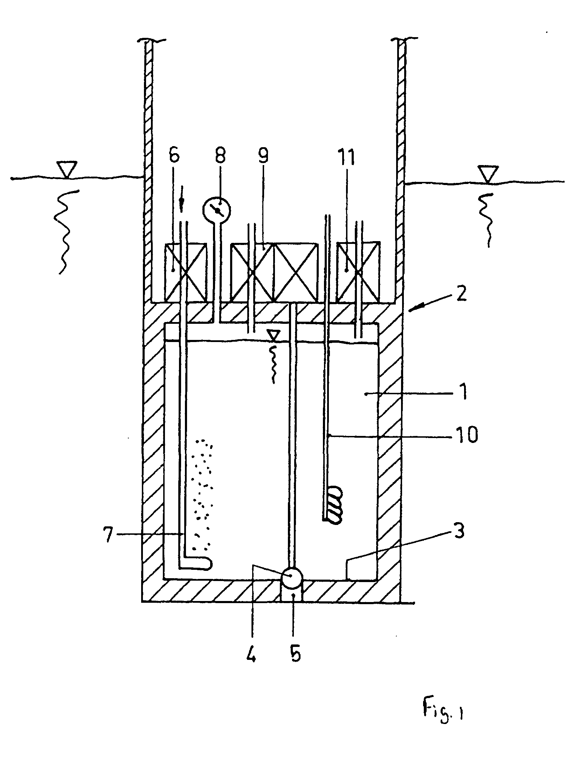

Nachfolgend wird ein Ausführungsbeispiel der Erfindung näher erläutert, das in der Zeichnung dargestellt ist.

Die Zeichnung zeigt in einem vereinfachten Längsschnitt eine Vorrichtung zur Bestimmung des Gehalts an oxidierbaren Inhaltsstoffen in wässrigen Flüssigkeiten, insbesondere des Gehalts an Kohlenstoff oder Stickstoff in Abwasser. Eine Probenkammer 1 ist in einer in das zu untersuchende Abwasser eintauchbaren Meßboje 2 angeordnet. Am Kammerboden 3 ist eine durch ein Ventil 4 verschließbare Kammeröffnung 5 angeordnet. Durch ein Gaseinlaßventil 6 und eine in die Probenkammer 1 ragende Gasleitung 7 kann Sauerstoff oder CO₂-freies Gas zugeführt werden. Als Druckmeßeinrichtung ist eine Druckmeßdose 8 an die Probenkammer 1 angeschlossen. Stattdessen kann auch eine Feuchtemeßeinrichtung vorgesehen werden. Ein Gasablaßventil 9 dient dazu, das Gas aus der Probenkammer 1 abzulassen. Als Heizelement ragt in die Probenkammer 1 ein Glühdraht 10. Eine Dosiereinrichtung 11 zur Zugabe von Säure zur Veränderung des pH-Wertes ist an die Probenkammer 1 angeschlossen.

Die als Reaktionsraum oder Verbrennungsofen dienende Probenkammer 1 wird bei geöffnetem Ventil 4 und offenem Gasauslaßventil 9 mit dem zu untersuchenden Abwasser befüllt, das durch den hydrostatischen Druck des die Meßboje 2 umgebenden Abwassers durch die Kammeröffnung 5 in die Probenkammer 1 einströmt. Sodann wird das Ventil 4 geschlossen. Unter Einsatz einer pH-Meßeinrichtung wird durch Zugabe von Säure über die Dosiereinrichtung 11 ein pH-Wert von beispielsweise 2-3 eingestellt. Sodann wird die Probenkammer 1 über das Gaseinlaßventil 6 begast.

Durch das eindringende Gas wird das in der Probenkammer 1 enthaltene Wasser bei offenem Ventil 4 durch die Kammeröffnung 5 verdrängt. Sodann werden das Ventil 4 am Kammerboden 3 und das Gaseinlaßventil 6 geschlossen.

Das Heizelement 10 ist mit einer geringen Menge des zu untersuchenden Abwassers benetzt. Das Heizelement 10 wird kurzzeitig auf 1000-1200° C aufgeheizt. Dabei verdunstet das am Heizelement 10 anhaftende Wasser schlagartig und erhöht den Druck im Probenraum 1. Zugleich werden die oxidierbaren Wasserinhaltsstoffe zu CO₂ oder NO verbrannt.

Der durch das Verdampfen entstehende Druckanstieg wird in der Druckmeßdose 8 gemessen. Stattdessen kann auch der Feuchteanstig im Gas gemessen werden. Daraus wird in einer (nicht dargestellten) Auswerteeinrichtung die Menge des verdampften Wassers bestimmt.

Durch Öffnen des Gaseinlaßventils 6 und des Gasablaßventils 9 wird das entstandene Verbrennungsgas CO₂ und/oder NO einer (nicht dargestellten) Einrichtung zur Infrarotmessung zugeführt und vermessen. Der so erhaltene Wert ergibt zusammen mit dem aus dem Druckanstieg oder dem Feuchteanstieg ermittelten Volumen der verdampften Probenmenge den Gehalt an oxidierbaren Inhaltsstoffen, beim beschriebenen Beispiel den Gehalt an Kohlenstoff und/oder Stickstoff.

Das Verfahren wurde am Beispiel einer in das Abwasser eintauchenden Meßboje beschrieben. Es versteht sich, daß das Verfahren auch bei einer anderen Ausführung der Vorrichtung vorteilhaft einsetzbar ist, beispielsweise wenn das zu untersuchende Abwasser der Probenkammer über eine Leitung und eine Pumpe zugeführt wird.

## Patentansprüche

1. Verfahren zur Bestimmung des Gehalts an oxidierbaren Inhaltsstoffen in wässrigen Flüssigkeiten, bei dem Flüssigkeitsproben einem Verbrennungsofen zugeführt und thermisch behandelt werden und der Inhaltsstoff zu einem gasförmigem Oxid verbrannt wird und in einer Probe des so erhaltenen Abgases der Gehalt an gasförmigem Oxid durch Infrarotmessung ermittelt wird, **dadurch gekennzeichnet, daß** eine an einer Heizfläche anhängende Flüssigkeitsprobe bei geschlossenem Verbrennungsofen verdampft wird, um die der Infrarotmessung zuzuführende Abgasprobe zu erhalten, und daß der bei der Verdampfung entstehende Druckanstieg im Verbrennungsofen oder der Feuchteanstieg im Gas gemessen und daraus die Probenmenge rechnerisch bestimmt wird.

2. Verfahren nach Anspruch 1, **dadurch gekennzeichnet, daß** der Verbrennungsofen mit der zu untersuchenden Flüssigkeit mindestens teilweise gefüllt und anschließend durch Gaszufuhr entleert wird.

3. Verfahren nach Anspruch 1, **dadurch gekennzeichnet, daß** die Heizfläche auf etwa 1000-1200° C erhitzt wird.

4. Vorrichtung zur Durchführung des Verfahrens nach einem der Ansprüche 1-3, mit einem Verbrennungsofen und einer nachgeschalteten Einrichtung zur Infrarotmessung, **dadurch gekennzeichnet, daß** der Verbrennungsofen eine abschließbare Probenkammer (1) aufweist, in die ein Heizelement (10) ragt und an die eine Druckmesseinrichtung (8) oder Feuchtemeßeinrichtung angeschlossen ist.

5. Vorrichtung nach Anspruch 4, **dadurch gekennzeichnet, daß** das Heizelement (10) einen Glühdraht aufweist.

6. Vorrichtung nach Anspruch 4, **dadurch gekennzeichnet, daß** die Probenkammer (1) in einer in die zu untersuchende Flüssigkeit eintauchbaren Meßboje angeordnet ist und am Kammerboden (3) eine durch ein Ventil (4) verschließbare Kammeröffnung (5) aufweist.

## Claims

1. Method for determining the level of oxidizable constituents in aqueous liquids, in which liquid samples are fed to a combustion furnace and heat-treated, and the constituent is burnt to form a gaseous oxide, and in a sample of the exhaust gas obtained in this way the level of gaseous oxide is determined by infrared measurement, **characterized in that** a liquid sample which is attached to a heating surface is evaporated with the combustion furnace closed, in order to obtain the exhaust-gas sample which is to be fed for infrared measurement, and **in that** the pressure increase in the combustion furnace which occurs during the evaporation or the increase in the humidity of the gas is measured, and the quantity of sample is determined by calculation from this information.

2. Method according to Claim 1, **characterized in that** the combustion furnace is at least partially filled with the liquid which is to be analysed and is then emptied by gas being supplied.

3. Method according to Claim 1, **characterized in that** the heating surface is heated to approximately 1000-1200°C.

4. Apparatus for carrying out the method according to one of Claims 1-3, having a combustion furnace and a downstream device for infrared measurement,
**characterized in that** the combustion furnace has a closable sample chamber (1), into which a heating element (10) projects and to which a pressure-measuring device (8) or humidity-measuring device is connected.

5. Apparatus according to Claim 4, **characterized in that** the heating element (10) has a glow wire.

6. Apparatus according to Claim 4, **characterized in that** the sample chamber (1) is arranged in a measuring buoy, which can be immersed in the liquid which is to be analysed, and at the chamber base (3) has a chamber opening (5) which can be closed off by a valve (4).

## Revendications

1. Procédé pour la détermination de la teneur en substances oxydables dans des liquides aqueux, avec lequel des échantillons de liquide sont acheminés à un four de combustion et subissent un traitement thermique et la substance est brûlée pour former un oxyde gazeux et la teneur en oxyde gazeux est déterminée par mesure aux infrarouges dans un échantillon des gaz d'échappement ainsi obtenus, **caractérisé en ce qu'**un échantillon liquide attaché à une surface chauffante est vaporisé lorsque le four de combustion est fermé afin d'obtenir l'échantillon de gaz d'échappement à acheminer à la mesure aux infrarouges et **en ce que** l'augmentation de pression dans le four de combustion ou l'augmentation du taux d'humidité dans le gaz, produite lors de la vaporisation, est mesurée et la quantité d'échantillon en est déduite par calcul.

2. Procédé selon la revendication 1, **caractérisé en ce que** le four de combustion est au moins partiellement rempli avec le liquide à analyser et il est ensuite vidé par amenée de gaz.

3. Procédé selon la revendication 1, **caractérisé en ce que** la surface chauffante est chauffée à environ 1000-1200°C.

4. Dispositif pour la mise en oeuvre du procédé selon l'une des revendications 1 à 3, comprenant un four de combustion et un appareil de mesure aux infrarouges monté à la suite, **caractérisé en ce que** le four de combustion présente une chambre à échantillons (1) obturable dans laquelle pénètre un élément chauffant (10) et à laquelle est raccordé un appareil de mesure de la pression (8) ou un appareil de mesure de l'humidité.

5. Dispositif selon la revendication 4, **caractérisé en ce que** l'élément chauffant (10) présente un fil incandescent.

6. Dispositif selon la revendication 4, **caractérisé en ce que** la chambre à échantillon (1) est disposée dans une bouée de mesure qui peut être plongée dans le liquide à analyser et présente sur le fond de la chambre (3) une ouverture de chambre (5) qui peut être fermée par une vanne (4).
